(19) Europäisches Patentamt
European Patent Office
Office européen des brevets

(11) **EP 4 478 073 A1**

(12) **EUROPEAN PATENT APPLICATION**

(43) Date of publication:
**18.12.2024  Bulletin 2024/51**

(21) Application number: **24179890.9**

(22) Date of filing: **04.06.2024**

(51) International Patent Classification (IPC):
**G01R 33/46** (2006.01)      **G01R 33/465** (2006.01)

(52) Cooperative Patent Classification (CPC):
**G01R 33/465; G01R 33/4625**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.06.2023   JP 2023097130**

(71) Applicants:
• **NIPPON MEDICAL SCHOOL FOUNDATION
Bunkyo-ku
Tokyo 113-8602 (JP)**
• **JEOL Ltd.
Akishima, Tokyo 196-8558 (JP)**

(72) Inventors:
• **KANAWAKU, Yoshimasa
Japan, 113-8602 (JP)**
• **HIRAKAWA, Keiko
Japan, 113-8602 (JP)**
• **NAKAO, Tomoki
Tokyo, 196-8558 (JP)**
• **YOSHIMURA, Hironobu
Tokyo, 196-8558 (JP)**

(74) Representative: **Boult Wade Tennant LLP
Salisbury Square House
8 Salisbury Square
London EC4Y 8AP (GB)**

(54) **DATA PROCESSING APPARATUS, AND SAMPLE EVALUATION METHOD**

(57)    A multivariable analyzer (18) executes multivariable analysis on a data set formed from a plurality of spectrograms acquired from a plurality of samples, and identifies a primary component of the data set, as a result of the multivariable analysis. Each spectrogram has a first coordinate system. A distribution generator (20) generates a loading distribution corresponding to the primary component, as a result of the multivariable analysis. A plot generator (22) generates a loading plot having a second coordinate system, based on the loading distribution. The second coordinate system is identical to the first coordinate system.

FIG. 1

## Description

TECHNICAL FIELD

The present disclosure relates to a technique for processing data generated through NMR (Nuclear Magnetic Resonance) measurement with an NMR apparatus.

BACKGROUND

Methods of evaluating a characteristic of a mixture are known. The mixture is, for example, a biological sample such as a serum, or a mixture solution sample including a polymer compound or the like.

Documents 1 and 2 describe methods of evaluating a characteristic of a biological sample. More specifically, Documents 1 and 2 describe methods including a step of acquiring an FID (Free Induction Decay) signal derived from a biological sample using an NMR apparatus, and calculating a spectrogram by repeating time-frequency analysis over the entirety of the FID signal. Documents 1 and 2 also describe methods including generating a score plot by executing multivariable analysis on a spectrogram, and identifying an attribute of a biological sample based on the score plot.

Documents 3 and 4 describe apparatuses which execute bucket integration using a bucket set on target spectrum data, to contract the target spectrum data into histogram data.

CITATION LIST

[Document 1] JP 2015-114157 A
[Document 2] JP 2019-158868 A
[Document 3] JP 5415476 B
[Document 4] JP 5020491 B

As an example of time-frequency analysis, a short-time Fourier transform (STFT) is known. One of parameters which must be set in the short-time Fourier transform is a frame length. In the short-time Fourier transform, time resolution and frequency resolution are in a tradeoff relationship with each other. In order to obtain a desired time resolution or a desired frequency resolution, an appropriate frame length must be set. Provision of information for setting the appropriate frame length to a user is desired.

In the techniques of Documents 1 and 2, although it is possible to identify the attribute of the sample from the score plot, it is not possible to feed a result of the evaluation of the score plot back to parameters for time-frequency analysis. In addition, in the techniques of Documents 1 and 2, it is not possible to investigate factors that cause differences in the attribute among a plurality of samples (for example, a component or a structure which affects the difference in the attribute) or the like. This is similarly applicable to the techniques of Documents 3 and 4.

An advantage of the present disclosure lies in providing a novel method for investigating a characteristic or the like of a sample from a spectrogram generated through NMR measurement.

SUMMARY

(1) According to one aspect of the present disclosure, there is provided a data processing apparatus comprising: acquisition means; analysis means, and generation means. The acquisition means acquires a plurality of spectrograms generated by executing NMR measurement on a plurality of samples. Each of the plurality of spectrograms has a first coordinate system with a time axis and a frequency axis. The analysis means executes multivariable analysis on a data set formed from the plurality of spectrograms, to identify a primary component of the data set and generate a loading distribution corresponding to the primary component. The loading distribution is formed from a plurality of loadings corresponding to a plurality of variables in the multivariable analysis. The generation means generates a loading plot having a second coordinate system with a time axis and a frequency axis, based on the loading distribution. The second coordinate system is identical to the first coordinate system.

According to the structure described above, by referring to or analyzing the loading plot, it is possible to easily identify a sample component that significantly contributes to the primary component in the time-frequency coordinate system, and a sample component that does not significantly contribute to the primary component. Based on the loading plot, a parameter set for time-frequency analysis may be corrected, or targets of the multivariable analysis may be narrowed down. A processor to be described later functions as the acquisition means, the analysis means, and the generation means. The processor further functions as some of the other means described below. A multivariable analyzer and a distribution generator to be described below correspond to the analyzing means.

In an embodiment, a particular spectrogram is selected from among the plurality of spectrograms. The data processing apparatus according to the embodiment further comprises display control means that causes the particular spectrogram and the loading plot to be displayed side by side on a display. According to this structure, comparison of the particular spectrogram to the loading plot can be facilitated.

A data processing apparatus of an embodiment further comprises setting means that sets, on the loading plot, an analysis region defined by at least one of a time range and a frequency range. The analysis means again executes (re-executes) the multivariable analysis on a limited data set corresponding to the analysis region. The data set normally includes a part that is important for uncovering a difference among a plurality of samples, and a part that is not important for this purpose. According to the structure described above, multivariable analysis can be executed on the important part in the data set while removing the non-important part in the data set.

In an embodiment, in the loading plot, the time range and the frequency range are designated by a user. The setting means sets the analysis region in accordance with the time range and the frequency range designated by the user.

In an embodiment, the setting means sets, as the analysis region, a region that belongs to the time range and the frequency range, and that satisfies a particular loading condition. The particular loading condition is satisfied, for example, when a loading at a coordinate of interest belongs to a particular loading range. In an embodiment, the generation means generates the loading plot through coordinate transformation of the loading distribution.

A data processing apparatus according to an embodiment further comprises: means that executes time-frequency analysis on a plurality of FID signals which are generated by executing the NMR measurement on the plurality of samples in accordance with a parameter set, to thereby generate the plurality of spectrograms; means that corrects the parameter set based on the analysis region; and means that re-executes the time-frequency analysis on the plurality of FID signals in accordance with the corrected parameter set, to thereby generate a plurality of spectrograms forming the limited data set. According to this structure, for example, the limited data set can be acquired under a desired time resolution or desired frequency resolution. The parameter set may include a frame length, a frame interval, or the like. Alternatively, the parameter set may be corrected by the user.

A data processing apparatus according to an embodiment further comprises means that cuts out a plurality of parts forming the limited data set from the plurality of spectrograms, based on the analysis region. According to this structure, it becomes possible to re-use the plurality of spectrograms.

According to another aspect of the present disclosure, there is provided a program for executing a data processing method on a computer. The data processing method comprises: an acquisition step in which a plurality of spectrograms generated by executing NMR measurement on a plurality of samples are acquired, each of the plurality of spectrograms having a first coordinate system with a time axis and a frequency axis; an analysis step in which multivariable analysis is executed on a data set formed from the plurality of spectrograms, to identify a primary component of the data set and generate a loading distribution corresponding to the primary component, the loading distribution being formed from a plurality of loadings corresponding to a plurality of variables in the multivariable analysis; and a generation step in which a loading plot having a second coordinate system with a time axis and a frequency axis is generated based on the loading distribution. The second coordinate system is identical to the first coordinate system. The data processing method described above corresponds to a sample evaluation method.

(2) According to another aspect of the present disclosure, there is provided a data processing apparatus comprising: acquisition means that acquires a plurality of spectrograms generated by executing NMR measurement on each of a plurality of samples, and defined by time and frequency; multivariable analysis means that executes multivariable analysis on the plurality of spectrograms, to generate a primary component for separating the plurality of samples based on sample attributes; distribution generation means that generates a loading distribution of a particular primary component corresponding to an index array determined by time information and frequency information included in the plurality of spectrograms, based on result of the multivariable analysis by the multivariable analysis means; and plot generation means that generates a loading plot by representing the loading distribution of the particular primary component on a coordinate system defined by time and frequency.

The data processing apparatus may further comprise display control means that displays a spectrogram of a designated sample and the loading plot side by side on a display.

The data processing apparatus may further comprise specifying means that specifies an analysis region by a time range and a frequency range on the loading plot, and the multivariable analysis means, the distribution generation means, and the plot generation means may execute respective processes targeted on the analysis region.

The specifying means may specify, when the time range and the frequency range are designated on the loading plot by a user, a region defined by the time range and the frequency range designated by the user as the analysis region.

The specifying means may specify, as the analysis region, a region defined by a time range and a frequency range, and in which a loading is in a particular range.

The plot generation means may generate the loading plot through coordinate transformation of the loading distribution.

According to another aspect of the present disclosure, there is provided a program which, when executed, causes a computer to function as: acquisition means that acquires a plurality of spectrograms generated through NMR measurement of each of a plurality of samples, and defined by time and frequency; multivariable analysis means that executes multivariable analysis on the plurality of spectrograms, to generate a primary component for separating the plurality of samples based on sample attributes; distribution generation means that generates a loading distribution of a particular primary component with respect to an index array determined by time information and frequency information included in the plurality of spectrograms, based on result of the multivariable analysis by the multivariable analysis means; and plot generation means that generates a loading plot by representing a loading distribution of the particular primary component on a coordinate system defined by time and frequency.

According to another aspect of the present disclosure, there is provided a method of evaluating a characteristic, the method comprising: a first step in which a plurality of spectrograms generated through NMR measurement on each of a plurality of samples, and defined by time and frequency are acquired; a second step in which multivariable analysis is executed on the plurality of spectrograms, to generate a primary component for separating the plurality of samples based on sample attributes; a third step in which a loading distribution of a particular primary component with respect to an index array determined by time information and frequency information included in the plurality of spectrograms is generated based on result of the multivariable analysis in the second step; and a fourth step in which a loading plot is generated by representing a loading distribution of the particular primary component on a coordinate system defined by time and frequency.

BRIEF DESCRIPTION OF DRAWINGS

Embodiment(s) of the present disclosure will be described based on the following figures, wherein:

FIG. 1 is a block diagram showing a structure of a data processing system according to an embodiment of the present disclosure;
FIG. 2 is a block diagram showing a structure of hardware of a data processing apparatus according to an embodiment of the present disclosure;
FIG. 3 is a diagram showing an FID signal;
FIG. 4 is a diagram showing a spectrogram of HSA;
FIG. 5 is a diagram showing a spectrogram of HDL;
FIG. 6 is a diagram showing a spectrogram of LDL;
FIG. 7 is a diagram showing a score plot;
FIG. 8 is a diagram showing a loading distribution;
FIG. 9 is a diagram showing a loading plot corresponding to a first primary component (PC-1);
FIG. 10 is a diagram for explaining coordinate transformation between data in a two-dimensional format and data in a one-dimensional format;
FIG. 11 is a diagram showing a spectrogram and a loading plot of HSA;
FIG. 12 is a diagram showing an analysis region which is set on the loading plot;
FIG. 13 is a diagram showing a score plot;
FIG. 14 is a diagram showing a loading plot;
FIG. 15 is a diagram showing a spectrogram generated from a serum of BKS18;

FIG. 16 is a diagram showing a spectrogram generated from a serum of Jcl;
FIG. 17 is a diagram showing a score plot;
FIG. 18 is a diagram showing a loading plot;
FIG. 19 is a diagram showing a score plot;
FIG. 20 is a diagram showing a loading plot;
FIG. 21 is a diagram showing a spectrogram generated from a serum of a PD patient;
FIG. 22 is a diagram showing a spectrogram generated from a serum of a non-PD patient;
FIG. 23 is a diagram showing a score plot;
FIG. 24 is a diagram showing a loading plot;
FIG. 25 is a diagram showing a loading plot; and
FIG. 26 is a diagram showing a data processing method according to an embodiment of the present disclosure.

DESCRIPTION OF EMBODIMENTS

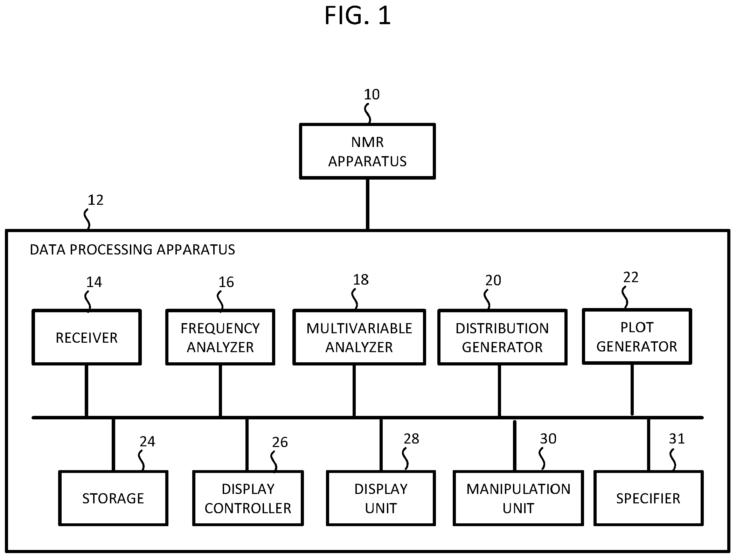

A data processing system according to an embodiment of the present disclosure will now be described with reference to FIG. 1. FIG. 1 is a block diagram showing a structure of a data processing system according to the embodiment. A data processing system according to the embodiment comprises an NMR apparatus 10 and a data processing apparatus 12.

The NMR apparatus 10 is an apparatus which illuminates a high frequency signal onto a sample placed in a static magnetic field, and detects a high frequency signal emitted from the sample. In the present embodiment, NMR measurement is executed by the NMR apparatus 10 on each of a plurality of different samples, to detect an FID signal of each of the plurality of samples. The FID signal has a waveform representing a change with respect to time of an amplitude of an observed high frequency signal.

Each sample is a mixture, and is, for example, a biological sample such as a serum, or a mixture solution sample including a polymer compound or the like. Alternatively, other mixtures may be used as the sample.

The data processing apparatus 12 is an apparatus which receives the FID signal detected by the NMR apparatus 10, and executes a process on the FID signal. The data processing apparatus 12 executes the process on the FID signal of each of the plurality of samples. The data processing apparatus 12 may be included in the NMR apparatus 10, may be a separate apparatus from the NMR apparatus 10 when physically viewed, or may be formed from a plurality of apparatus which are physically distanced away from each other.

For example, the data processing apparatus 12 includes a receiver 14, a frequency analyzer 16, a multivariable analyzer 18, a distribution generator 20, a plot generator 22, a storage 24, a display controller 26, a display unit 28, a manipulation unit 30, and a specifier 31.

The receiver 14 receives the FID signal of each sample detected by the NMR apparatus 10. For example, the receiver 14 may receive the FID signal of each sample from the NMR apparatus 10 through wired communication or wireless communication. As another example, the FID signal of each sample may be stored in a storage device such as a memory or a hard disk drive, and may be input to the data processing apparatus 12 via the storage device, and the receiver 14 may receive the FID signal which is input. The receiver 14 corresponds to an acquisition portion or acquisition means.

The frequency analyzer 16 generates a spectrogram of each of the plurality of samples by repeating time-frequency analysis on the FID signal of each of the plurality of samples. For example, as the time-frequency analysis, short-time Fourier transform (STFT) is used. The spectrogram is an image representing time, frequency, and intensity. The frequency analyzer 16 calculates a frequency spectrum by executing frequency analysis while applying a window function on the FID signal acquired from each sample, at each time on a time axis. Each sample is a mixture sample, and each FID signal is a composite signal formed from a plurality of FID signal components corresponding to the plurality of sample components. The frequency analyzer 16 generates the spectrogram based on a plurality of frequency spectra on the time axis. For example, the spectrogram is generated by representing the intensity (amplitude) of each individual frequency component by color or brightness on a two-dimensional coordinate system (first coordinate system) defined by a time axis and a frequency axis. The spectrogram is data in a two-dimensional format, defined by time and frequency.

Alternatively, the spectrogram of each sample may be generated by the NMR apparatus 10. In this case, the receiver 14 receives the spectrogram of each sample.

The multivariable analyzer 18 executes multivariable analysis on a data set formed from a plurality of spectrograms corresponding to the plurality of samples. For example, the multivariable analysis is principal component analysis (PCA), PLS discriminant analysis (PLS-DA), or soft independent modeling of class analogy (SIMCA). For example, two combined variables are generated. No particular limitation is imposed on the number of combined variables to be generated.

Each spectrogram corresponds to a two-dimensional intensity matrix. The intensity matrix is specifically formed from N columns arranged in a direction of the time axis (horizontal axis), and each column is formed from M intensities arranged in a direction of the frequency axis (vertical axis). (MxN) intensities forming each spectrogram will hereinafter be called a sub data set. When the number of samples which are the analysis targets is S, the data set is formed from S sub data sets. The data set is formed from (SxMxN) intensities. Each individual intensity is specified by time, frequency, and sample number. In actual practice, each individual intensity is treated as data of a vector format.

For example, when a two-dimensional data set is considered as the data set, the data set has an index axis and a sample axis. The index axis is an axis representing (MxN) indices indicating (MxN) variables (more specifically, intensities). The index corresponds to a combination of time and frequency. The sample axis is an axis representing S samples.

In the embodiment, the multivariable analyzer 18 executes primary component analysis on the data set formed in a manner described above. The primary component analysis is a method of putting together data in a high dimensional space into a lower dimensional space. In the embodiment, in the primary component analysis, a primary component which maximizes variance of the plurality of spectrograms; that is, a primary component which remarkably expresses a difference among the plurality of samples, is searched for. In reality, the search is repeated. With this process, in the embodiment, a first primary component and a second primary component are identified. Alternatively, more primary components may be identified. In the embodiment, as a result of the primary component analysis, a score for the first primary component and a score for the second primary component are calculated for each spectrogram; that is, for each sample. The score for the first primary component corresponds to a position on a first primary component axis. Similarly, the score for the second primary component corresponds to a position on a second primary component axis. Each primary component is defined by a linear equation including a large number of variables. In the linear equation, a coefficient applied to each variable is called a "loading" (or a "loading value"). The loading of each variable for a certain primary component represents a degree of contribution (that is, weight) of each variable for the primary component. The multivariable analyzer 18 plots, for example, the plurality of scores corresponding to the plurality of samples on a two-dimensional coordinate system having the first primary component axis and the second primary component axis, to generate a score plot.

The distribution generator 20 generates, for each primary component, a loading distribution corresponding to the primary component, based on result of the multivariable analysis or as the result of the multivariable analysis. In the embodiment, the loading distribution is a one-dimensional array of numerical values, formed from (MxN) loadings corresponding to (MxN) variables. Because (MxN) variables are identified by (MxN) indices, it can be alternatively understood that the loading distribution is formed from (MxN) loadings corresponding to (MxN) indices. Alternatively, the distribution generator 20 may be incorporated in the multivariable analyzer 18.

The plot generator 22 generates a loading plot as a two-dimensional graph, based on the loading distribution (data in the one-dimensional format) corresponding to a particular primary component. The particular primary component is typically the first primary component. Alternatively, the particular primary component may be selected by a user. More specifically, the loading plot has a coordinate system (second coordinate system) having a time axis and a frequency axis. The plot generator 22 plots each loading forming the loading distribution on the coordinate system. In this process, a coordinate on which each loading is to be plotted is specified from the index corresponding to each loading. A magnitude of the loading is represented with color or brightness. In the embodiment, the second coordinate system of the loading plot is identical to the first coordinate system of the spectrogram. That is, the second coordinate system is matched with the first coordinate system so as to enable concrete evaluation of the content of the spectrogram while referring to the loading plot. According to the embodiment, the spectrogram and the loading plot can be easily compared with each other. The loading plot according to the embodiment differs from a typical loading plot which has a coordinate system with two primary component axes, and is a special loading plot (TF-loading plot) having a time-frequency coordinate system. In the loading plot, a change with respect to time of the loading is represented for each frequency or each frequency band. Such a change with respect to time does not occur in a general loading plot of the related art.

The storage 24 is realized by a storage device. For example, the storage 24 stores the FID signal, the spectrogram data, the result of multivariable analysis (for example, a plurality of scores, a plurality of loadings, etc.), the loading distribution data, the loading plot, or the like.

The display controller 26 controls display of each piece of information. For example, the display controller 26 causes the FID signal, the spectrogram, the result of multivariable analysis, the loading distribution, the loading plot, or the like to be displayed on the display unit 28.

In the present embodiment, the display controller 26 displays the spectrogram and the loading plot side by side on the display unit 28. For example, when a spectrogram to be displayed is designated by the user, the display controller 26 causes the designated spectrogram and the loading plot to be displayed side by side on the display unit 28.

The display unit 28 is a display such as a liquid crystal display, an EL display, or the like. The manipulation unit 30 is an inputting device such as a keyboard, a mouse, an input key, a manipulation panel, or the like.

The specifier 31 functions as a setter. More specifically, the specifier 31 sets a region to be analyzed (hereinafter referred to as "analysis region"). For example, the specifier 31 sets, on the loading plot, an analysis region defined by a time range and a frequency range. For example, when the user designates a time range and a frequency range on the loading plot, the specifier 31 sets a two-dimensional region defined by the time range and the frequency range designated by the user as the analysis region. As an alternative example, the specifier 31 may set, as the analysis region, a region which belongs to the time range and the frequency range, and which satisfies a particular loading condition. The particular loading condition is satisfied, for example, when a loading at a coordinate of interest belongs to a particular loading range. The particular loading range is, for example, a range of greater than or equal to a threshold, a range of greater than or equal to a lower limit value and lower than an upper limit value, or the like. The particular loading range may be designated by the user, or may be defined in advance. For example, the specifier 31 specifies as the analysis region a region in the loading plot having a loading of greater than or equal to a threshold. The specifier 31 does not need to be included in the data processing apparatus 12.

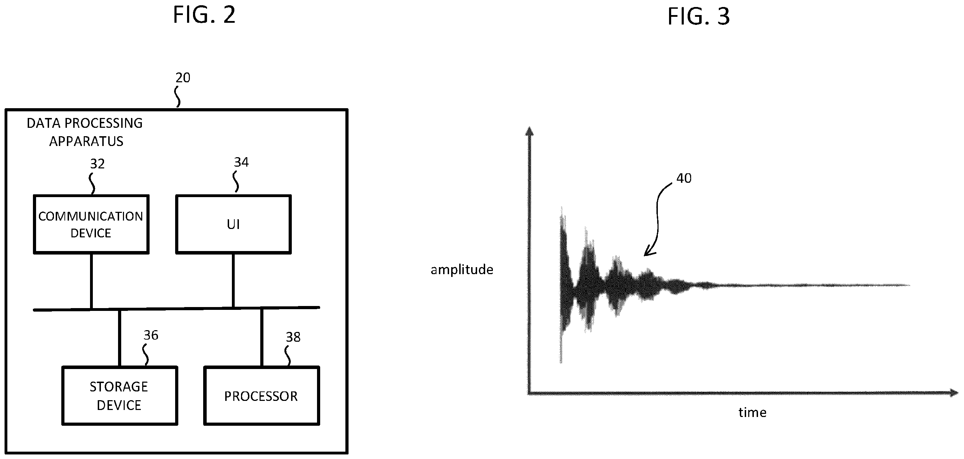

A structure of hardware of the data processing apparatus 12 will now be described with reference to FIG. 2. FIG. 2 is a block diagram showing a structure of the hardware of the data processing apparatus 12.

For example, the data processing apparatus 12 comprises a communication device 32, an UI (User Interface) 34, a storage device 36, and a processor 38.

The communication device 32 includes one or a plurality of communication interfaces having a communication chip, a communication circuit, or the like, and has a function to transmit information to other devices, and a function to receive information from other devices. The communication device 32 may have a wireless communication function or a wired communication function.

The UI 34 is a user interface, and includes a display and an inputting device. The display is a liquid crystal display, an EL display, or the like. The inputting device is a keyboard, a mouse, an input key, a manipulation panel, or the like. The display unit 28 and the manipulation unit 30 are realized by the UI 34. The UI 34 may be a UI such as a touch panel which has functions of both the display and the inputting device.

The storage device 36 is a device which forms one or a plurality of storage regions for storing data. The storage device 36 is, for example, a hard disk drive (HDD), a solid state drive (SSD), any of various memories (for example, a RAM, a DRAM, an NVRAM, a ROM, or the like), other storage devices (for example, an optical disk), or a combination of these. The storage 24 is realized by the storage device 36.

The processor 38 controls operations of various components of the data processing apparatus 12. The frequency analyzer 16, the multivariable analyzer 18, the distribution generator 20, the plot generator 22, the display controller 26, and the specifier 31 are realized by the processor 38. The storage device 36 may be used for realizing these components.

For example, the processor 38 is formed from a CPU (Central Processing Unit), a GPU (Graphics Processing Unit), an ASIC (Application Specific Integrated Circuit), an FPGA (Field Programmable Gate Array), a DSP (Digital Signal Processor), other programmable logic devices, an electronic circuit, or the like.

Alternatively, the functions of the data processing apparatus 12 may be realized by cooperation of a hardware resource and a software resource. For example, the functions are realized by the CPU forming the processor 38 reading and executing a program stored in the storage device 36. The program is stored in the storage device 36 via a recording medium such as a CD and a DVD, or via a communication path such as a network. As another example, the functions of the data processing apparatus 12 may be realized by a hardware resource such as an electronic circuit.

Processing by the data processing apparatus 12 will now be described in detail.

FIG. 3 shows an example of the FID signal. An FID signal 40 is an FID signal for a certain sample. The FID signal 40 is a signal detected by the NMR apparatus 10, and has a waveform representing a change with respect to time of an amplitude of an observed high frequency signal. When the receiver 14 receives the FID signal 40, the frequency analyzer 16 executes the short-time Fourier transform on the FID signal 40, to generate the spectrogram.

Here, as an example, an aqueous solution of a serum standard substance is used as the sample. More specifically, three samples are used, including HSA (Human Serum Albumin solution), HDL (Lipoproteins, High Density, Human Plasma) and LDL (Lipoproteins, Low Density, Human Plasma).

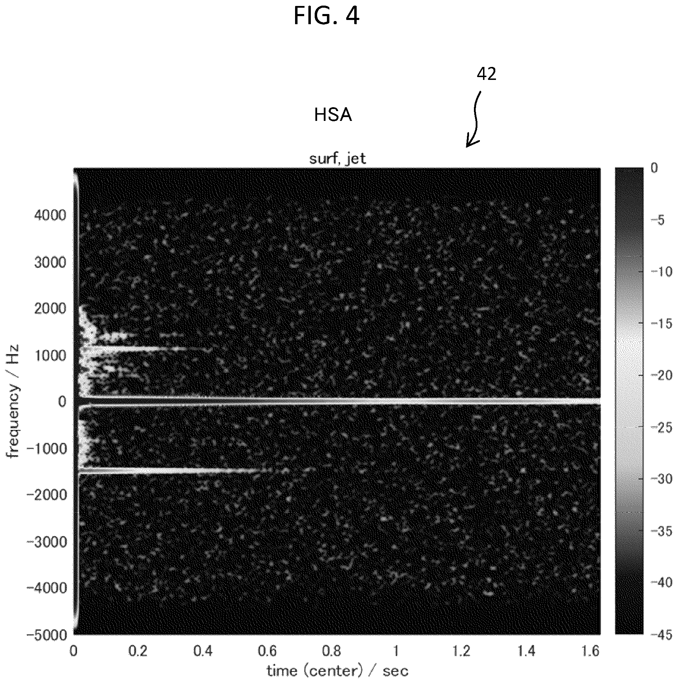

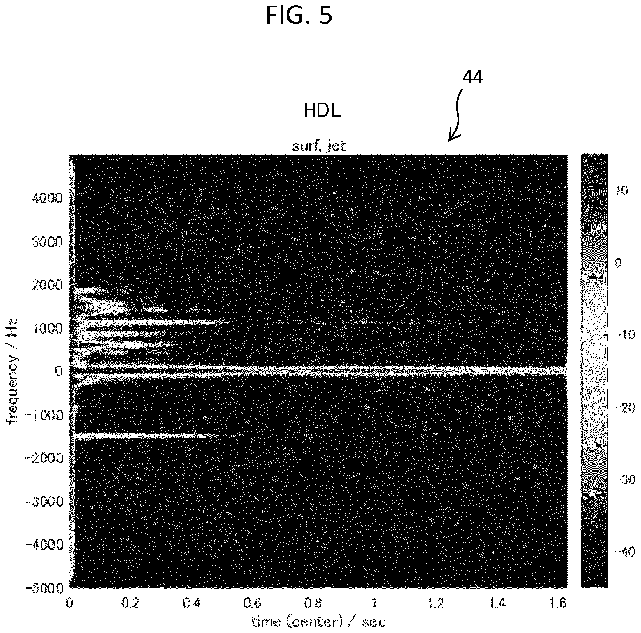

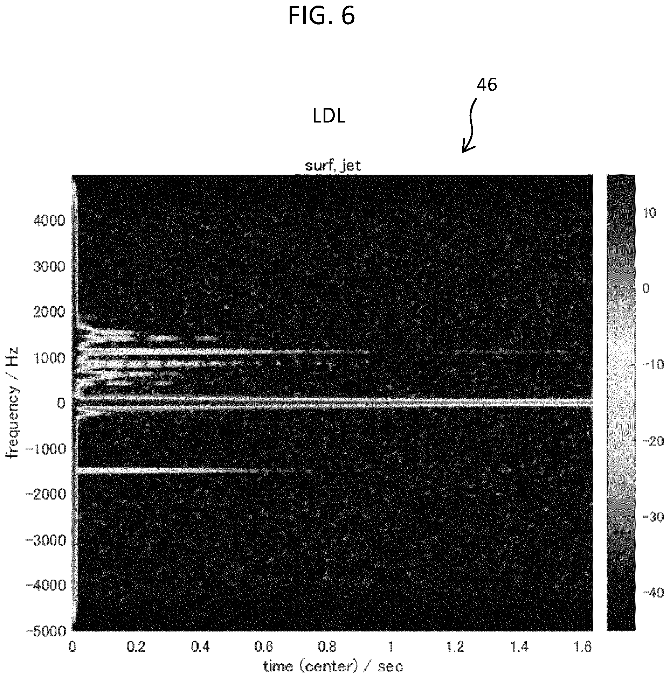

FIG. 4 shows a spectrogram 42 of HSA. FIG. 5 shows a spectrogram 44 of HDL, and FIG. 6 shows a spectrogram 46 of LDL.

When HSA, HDL, and LDL are measured by the NMR apparatus 10, FID signals respectively for HSA, HDL, and LDL are detected. The receiver 14 receives the FID signals respectively for HSA, HDL, and LDL. The frequency analyzer 16 executes the short-time Fourier transform on each of the FID signals for HSA, HDL, and LDL, to generate a spectrogram for each of HSA, HDL, and LDL.

In the spectrograms 42, 44, and 46, the horizontal axis shows the time, and the vertical axis shows the frequency. The intensity of each individual frequency component is represented by color (or brightness). A characteristic of the sample can be represented by the spectrogram. A color bar is shown at the right of each of the spectrograms 42, 44, and 46. Because each sample is formed from a plurality of sample components, on each of the spectrograms 42, 44, and 46, a stripe pattern can be observed. More specifically, each of the spectrograms 42, 44, and 46 has a plurality of streaks arranged in the direction of the frequency axis. Each streak is parallel to the direction of the horizontal axis, and shows an attenuation characteristic of the FID signal component.

The multivariable analyzer 18 executes multivariable analysis on a data set formed from the spectrograms 42, 44, and 46, to generate a plurality of combined variables. Here, as an example, the multivariable analyzer 18 executes the primary component analysis, to identify a first primary component (PC-1) and a second primary component (PC-2), and calculates two loading distributions corresponding to the two primary components and a score of each sample with respect to each primary component.

More specifically, when the number of samples is S, the multivariable analyzer 18 represents in a vector format multiple pieces of data (intensities) forming the S spectrograms, and executes the primary component analysis on (SxMxN) pieces of data (that is, data set) represented in the vector format. Through the primary component analysis, the loading distribution is generated for each primary component. The loading distribution is formed from (MxN) loadings represented in the vector format.

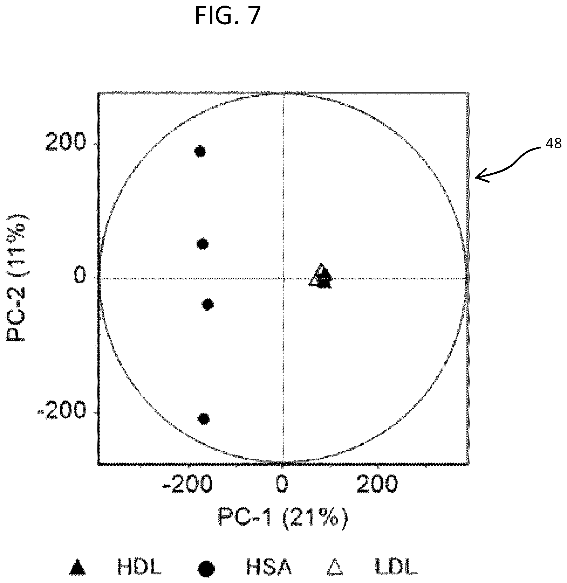

The multivariable analyzer 18 identifies, for each sample, a coordinate corresponding to the score of the sample (more specifically, the first primary component score and the second primary component score) on a two-dimensional coordinate system defined by a first primary component (PC-1) axis and a second primary component (PC-2) axis, and plots a display element on the coordinate, to thereby generate a score plot. FIG. 7 shows an example of the score plot.

In a score plot 48 shown in FIG. 7, the horizontal axis shows the first primary component (PC-1), and the vertical axis shows the second primary component (PC-2). Here, as an example, a contribution percentage of the first primary component (PC-1) is 21%, and a contribution percentage of the second primary component (PC-2) is 11%. The contribution percentage is an indication showing a degree of contribution of the primary component with respect to the data as a whole. The user can empirically recognize importance of the primary component by referring to the contribution percentage. Black circle marks show the scores of HSA. Black triangle marks show the scores of HDL, and white triangle marks show the scores of LDL.

The user can recognize a degree of separation of the plurality of samples by referring to the score plot 48. When a distance between a plurality of plots is short on the score plot 48, the plurality of samples can be evaluated as having similar characteristics. On the other hand, when the distance between a plurality of plots is long, the plurality of samples can be evaluated as having different characteristics. In the example configuration of FIG. 7, the marks showing HSA are distributed on the score plot 48 while being distanced from the marks showing HDL and the marks showing LDL. Because of this, it can be evaluated on the score plot 48 that HSA, HDL, and LDL are well separated from each other. When a degree of variance of the score is large for a certain primary component, the primary component can be evaluated as significantly contributing to the separation of the plurality of samples.

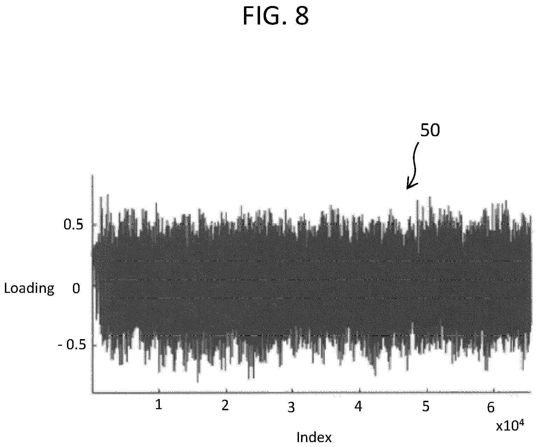

The distribution generator 20 generates a loading distribution for a particular primary component based on result of primary component analysis by the multivariable analyzer 18. Here, as an example, the distribution generator 20 generates the loading distribution for the first primary component (PC-1). FIG. 8 shows a loading distribution 50. The horizontal axis shows the index, and the vertical axis shows the loading for the first primary component (PC-1). The index corresponds to a combination of time and frequency.

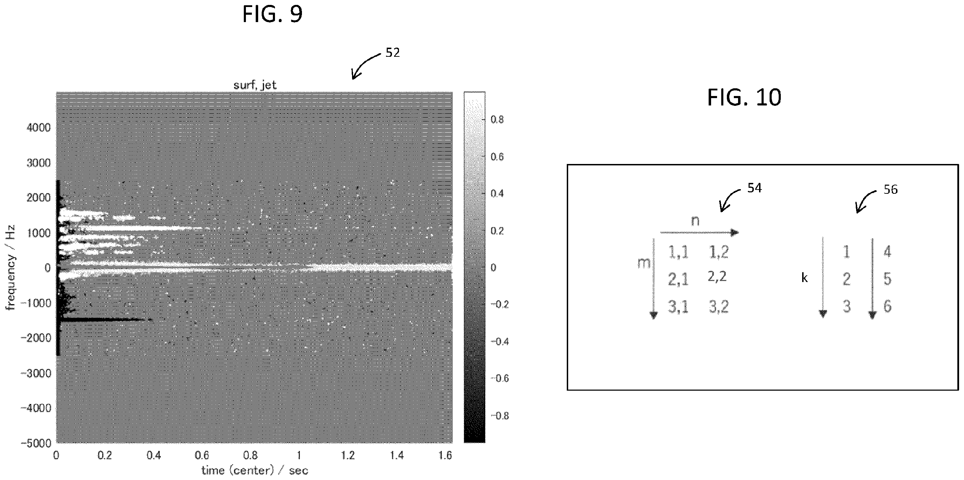

The plot generator 22 generates a loading plot based on the loading distribution 50, and by representing each loading with color or brightness on a two-dimensional coordinate system defined by a time axis and a frequency axis. FIG. 9 shows a loading plot 52. The plot generator 22 more specifically generates the loading plot 52 through coordinate transformation of the loading distribution 50 having a one-dimensional data format into a loading distribution having a two-dimensional data format.

On the loading plot 52, the horizontal axis shows the time, and the vertical axis shows the frequency. A magnitude of the loading for the first primary component (PC-1) is represented with color or brightness. The time axis in the loading plot 52 corresponds to the time axis in the spectrogram, and the frequency axis in the loading plot 52 corresponds to the frequency axis in the spectrogram.

With reference to FIG. 10, coordinate transformation from two-dimensional data into one-dimensional data, and coordinate transformation from the one-dimensional data to the two-dimensional data will now be described. As described above, the spectrogram is two-dimensional data. On the other hand, the data which is input to the multivariable analyzer 18 and the data which is output from the multivariable analyzer 18 are one-dimensional data. Therefore, the two-dimensional spectrogram must be converted to one-dimensional data before the multivariable analysis is executed. In addition, in order to generate the two-dimensional loading plot, the loading distribution which is one-dimensional data must be converted to two-dimensional data.

FIG. 10 shows a specific example of coordinate transformation. Reference numeral 54 shows two-dimensional data, and reference numeral 56 shows one-dimensional data.

In the two-dimensional data 54, a position in a vertical direction is represented by m, and a position in a horizontal direction is represented by n. A position in the one-dimensional data 56 is represented by k.

In the two-dimensional data 54, (n, m) shows a position on the two-dimensional space, or a value at this position. For example, (1, 1) shows the position (1, 1) on the two-dimensional space, or a value at the position (1, 1). In the example configuration of FIG. 10, m is a numerical value from 1 to 3, and n is 1 or 2.

In the one-dimensional data 56, k shows a position on the one-dimensional space, or a value at this position. For example, (1) shows a position (1) on the one-dimensional space, or a value at the position (1).

A relationship between m, n, and k can be represented by following Equation (1).

$$k = m + (n-1) \times M \quad (1)$$

Here, M is a number of positions in the vertical direction in the two-dimensional data. In the example configuration of FIG. 10, M is 3 (M=3). For example, when m=1 and n=1, k is 1 (k=1).

According to the relationship defined by Equation (1) described above, the two-dimensional data is converted into the one-dimensional data, and the one-dimensional data is converted into the two-dimensional data.

For example, when the primary component analysis is executed on a two-dimensional spectrogram, the multivariable analyzer 18 converts the spectrogram which is two-dimensional data into one-dimensional data (sub data set) for each sample, according to the above-described relationship. The multivariable analyzer 18 executes the primary component analysis on the data set which is a collected group of a plurality of pieces of one-dimensional data corresponding to a plurality of samples. The distribution generator 20 generates the loading distribution for each primary component, as a result of the primary component analysis. In the embodiment, among a plurality of loading distributions corresponding to a plurality of primary components, typically, a loading distribution corresponding to the first primary component is used. That is, the plot generator 22 converts the loading distribution corresponding to the first primary component (one-dimensional data) into the loading plot (two-dimensional data) according to the above-described relationship.

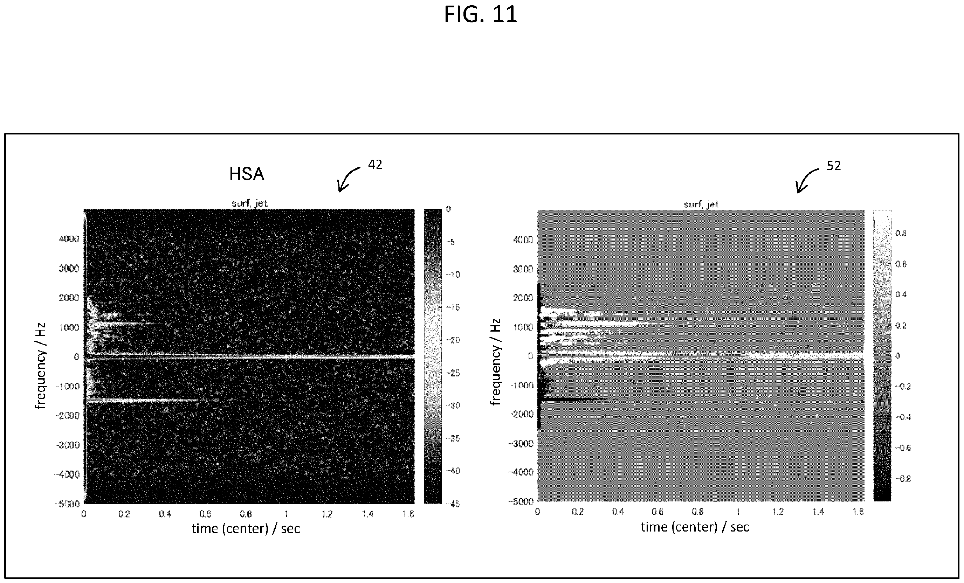

The display controller 26 causes the spectrogram and the loading plot to be displayed side by side on the display unit 28. For example, when the user designates, using the manipulation unit 30, the spectrogram 42 for HSA as a comparison target, and instructs comparative display, the display controller 26 causes the spectrogram 42 and the loading plot 52 to be displayed side by side on the display unit 28, as shown in FIG. 11.

In a manner described above, the loading distribution 50 is converted to the loading plot 52. The loading plot 52 is a two-dimensional image having a time axis serving as the horizontal axis and a frequency axis serving as the vertical axis. In the loading plot 52, each loading is represented with color or brightness. The horizontal axis of the loading plot 52 is the time axis, similar to the horizontal axis of the spectrogram 42, and the vertical axis of the loading plot 52 is the frequency axis, similar to the vertical axis of the spectrogram 42. On the spectrogram 42, an intensity of a frequency component is represented with color or brightness, and, on the loading plot 52, the loading is represented with color or brightness. The second coordinate system of the loading plot 52 is the same coordinate system as the first coordinate system of the spectrogram 42, and, thus, the user can easily compare the spectrogram 42 and the loading plot 52.

In the example display shown in FIG. 11, the spectrogram 42 and the loading plot 52 are displayed side by side, but this display is merely exemplary. Alternatively, the display controller 26 may cause the spectrogram 44 or the spectrogram 46 to be displayed side by side with the loading plot 52 on the display unit 28, in place of the spectrogram 42. For example, when the user designates the spectrogram 44 or the spectrogram 46 as the comparison target, the display controller 26 causes the designated spectrogram and the loading plot 52 to be displayed side by side on the display unit 28.

Alternatively, the display controller 26 may cause a plurality of spectrograms and the loading plot 52 to be displayed side by side on the display unit 28. For example, when the user designates the spectrograms 44 and 46, the display controller 26 causes the spectrograms 44 and 46 and the loading plot 52 to be displayed side by side on the display unit 28. In this manner, the user can easily compare the plurality of spectrograms and the loading plot.

The user can perform various investigations based on the two-dimensional loading distribution represented as the loading plot 52. For example, the user can perform various investigations based on a change of the loading in the frequency axis direction or the time axis direction (for example, change in color or brightness), based on a position of the frequency axis and/or on the time axis where a characteristic part exists, based on a mutual relationship among a plurality of positions where a plurality of characteristic parts exist, or the like.

In addition, the user can perform various investigations by comparing the two displayed items; that is, the spectrogram and the loading plot. For example, the user may specify a position on the frequency axis of a part with a high intensity on the spectrogram, and may observe, on the loading plot, what local state is being caused at the specified position on the frequency axis. Inversely, the user may specify a position (for example, a position on the frequency axis) on the loading plot with a high or low loading, and may observe, on the spectrogram, what local state is being caused at the specified position (for example, position on the frequency axis). This is similarly applicable to the position on the time axis. In addition to the above, the user may observe a same frequency range on the spectrogram and the loading plot, to compare the change of intensity and the change of loading, or may observe a same time range, to compare the change of intensity and the change of loading. Because the spectrogram and the loading plot have the same coordinate system, these comparisons can be easily made.

In the spectrogram, a position on the frequency axis where the intensity becomes high differs depending on the component included in the sample and the structure of the component. In general, in a spectrogram generated from a known sample, it is known what intensity distribution appears in what frequency region. A component included in the sample can be identified by reference to the intensity distribution in the spectrogram. In addition, because an attenuation rate (change with respect to time) of the NMR signal differs depending on the property of the component, the position of the intensity distribution on the time axis differs depending on the property of the component included in the sample.

The user can investigate a difference of the component or a difference of the structure which affects a difference in the attribute among a plurality of samples, by referring to a distribution form (for example, a change of loading in the frequency axis direction, a characteristic part on the frequency axis, or the like) on the loading plot, and comparing the distribution form with a distribution form on the spectrogram (for example, a change of the intensity in the frequency axis direction, a characteristic part on the frequency axis, or the like).

For example, the user may evaluate a characteristic of each component forming the sample, or analyze or investigate factors (for example, the component and the structure of the sample) that cause a change of the position or a difference of position on the frequency axis on the loading plot, by comparing the attenuation characteristic of each component on the spectrogram and the loading distribution on the loading plot.

For example, the user specifies a position on the frequency axis with a high intensity on the spectrogram. In general, it is known what intensity distribution appears in what frequency region. Because of this, by specifying the position on the frequency with a high intensity, it is possible to identify a component included in the sample. In the loading plot, when the loading is high at a particular position on the frequency axis, the component corresponding to the particular position can be deduced to be significantly contributing to the separation of the plurality of samples. That is, the component is deduced to have a high contribution percentage, for separating the plurality of samples. In this manner, a component which may contribute to separation of the plurality of samples may be deduced by comparing the spectrogram and the loading plot.

Alternatively, the analysis result of the loading plot may be fed back to the time-frequency analysis by the frequency analyzer 16. For example, when a characteristic loading distribution is caused at a certain position on the frequency axis on the loading plot (for example, when a high or low loading distribution occurs locally), it may be desired to increase the frequency resolution, to enable more detailed investigation of the characteristic loading distribution. In this case, a value which can increase the frequency resolution is set as the frame lengths of the short-time Fourier transform, and the short-time Fourier transform is executed on each FID signal. In this manner, the frequency resolution of the spectrogram corresponding to each sample can be increased. Then, through the primary component analysis of the plurality of spectrograms corresponding to the plurality of samples, the score plot and the loading plot are generated. By investigating the loading plot, it is possible to judge whether more importance should be placed on the frequency resolution or on the time resolution in the short-time Fourier transform. Alternatively, other parameters or the like in the time-frequency analysis (for example, the frame interval, and a window function form) may be changed based on the loading plot. When the parameter set for the time-frequency analysis is changed, the time-frequency analysis is re-executed on the plurality of FID signals which are already acquired, in accordance with the changed parameter set, so that a plurality of spectrograms are generated. The primary component analysis is re-executed on the data set formed from the plurality of spectrograms, and, as a result of the primary component analysis, a score plot and a loading plot are again generated.

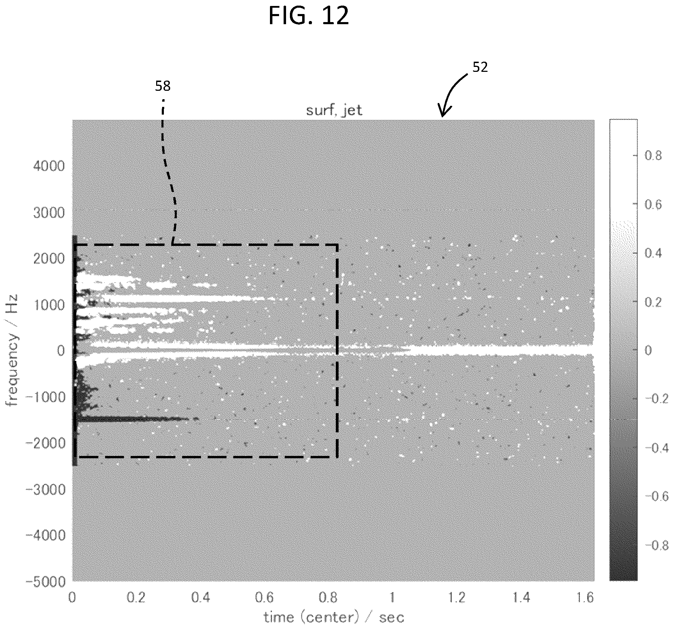

Next, processing by the specifier 31 will be described with reference to FIG. 12. FIG. 12 shows the loading plot 52.

For example, the loading plot 52 is displayed on the display unit 28. The user designates a time range and a frequency range on the loading plot 52 by manipulating the manipulation unit 30. The specifier sets as an analysis region a region 58 defined by the time range and the frequency range designated by the user.

When the analysis region is set, the frequency analyzer 16 re-executes the short-time Fourier transform on a part or a component corresponding to the analysis region in each FID signal, so that a limited spectrogram is generated. The multivariable analyzer 18 executes the multivariable analysis on a plurality of limited spectrograms (limited data set) corresponding to the plurality of samples. The distribution generator 20 generates a loading distribution based on the result of the multivariable analysis, and the plot generator 22 generates a loading plot based on the loading distribution. In this manner, a loading plot corresponding to the analysis region is generated. The loading plot can be called a limited loading plot. Desirably, the parameter set for short-time Fourier transfer is changed prior to the re-execution of the short-time Fourier transform.

For example, the user designates as the analysis region a region deduced to be contributing to the separation of the plurality of samples, by referring to the loading plot 52. That is, the user designates the analysis region, excluding a region deduced to be not contributing to the separation of the plurality of samples. With this process, the result of the primary component analysis can be improved.

In the example configuration of FIG. 12, the analysis region is specified by the region 58 of a quadrangular shape, but alternatively, the analysis region may be specified by a region of a shape other than the quadrangle (for example, a region of a shape of a circle, an ellipse, or any other arbitrary shape). For example, the user may designate the analysis region so as to include one or a plurality of characteristic parts.

The specifier 31 may specify, as the analysis region, a region which satisfies a loading condition. For example, the loading condition is satisfied when the loading is within a particular range (for example, a range of greater than or equal to a threshold, a range or less than a threshold, or a range of greater than or equal to a lower limit value and less than an upper limit value). For example, in the example configuration illustrated in FIG. 12, the specifier 31 may specify an analysis region to surround a part having a loading of greater than or equal to a threshold.

After the analysis region is set, if the change of the parameter set for the time-frequency analysis is not necessary, a plurality of parts corresponding to the analysis region may be cut out from the plurality of spectrograms which are already generated, without re-executing the time-frequency analysis. The multivariable analyzer 18 executes the primary component analysis on a limited data set formed from the plurality of cut-out parts. As a result of the primary component analysis, a new score plot and a new loading plot are generated. The cutting-out of the plurality of parts may be executed by the specifier 31 or the multivariable analyzer 18.

Some Examples of the present disclosure will now be described.

Example 1

Samples in Example 1 are HSA, HDL, and LDL. An objective of Example 1 is executing NMR measurement on albumin and lipoprotein within a human serum, and visualizing a difference between time-frequency characteristics of a plurality of NMR signals acquired from these samples.

Details of HSA, HDL, and LDL are shown below.

- HSA (Human Serum Albumin solution)

    Manufacturer: NMIJ
    Lot: 144
    Material number: NMIJ CRM 6202-a

- HDL (Lipoproteins High Density, Human Plasma)

    Manufacturer: Calbiochem
    Batch number: 3816722
    Material number: 437641-10MG

- LDL (Lipoproteins, Low Density, Human Plasma)

    Manufacturer: Calbiochem
    Batch number: 3883470&3912892
    Material number: 437644-10MG

(1) Apparatus Structure

As the NMR apparatus 10, JNM-ECZ400R manufactured by JEOL Ltd. was used. As software for data processing and controlling in the NMR apparatus 10, DELTA software (version 5.3.2 (JEOL Ltd.)) was used. In the data processing apparatus 12, an application program (hereinafter referred to as an "STFT tool") developed for MATLAB™ (The MathWorks, Inc.), and Unscrambler X version 11 (manufactured by Camo Software) were installed.

(2) Sample Creation and Measurement

For each of the samples described above, 100 μL of the sample was mixed with 500 μL of deuterated water (heavy water, manufactured by Sigma-Aldrich), and a sample solution thus generated was injected into an NMR sample tube having an outer diameter of 5 mm. A probe temperature at the NMR apparatus 10 was set at 30°C. 1H-NMR measurement was executed on the three kinds of samples described above, and FID signals for the three kinds of samples were detected. A resonance frequency of a signal derived from light water existing in each sample solution was set as an observation center frequency. With the use of the DANTE (Delays Alternating with Nutation for Tailored Excitation) pulse method, which is an example of a pulse program, as the measurement method, the signal derived from the light water was suppressed. This configuration facilitated the later analysis process, and results with superior quality were obtained. Depending on an objective of measurement, a plurality of sample tubes storing the same sample may be prepared, or a plurality of sample tubes storing a plurality of sample solutions having different dilution concentrations may be prepared.

(3) Analysis

(3-1) Time-Frequency Analysis

The STFT tool was started up, the FID signals of the plurality of samples acquired through the measurement were read into the tool, a sampling frequency and a data point number were input to the tool, and then, time-frequency analysis was executed on each of the FID signals. The spectrogram 42 illustrated in FIG. 4 is an example of the spectrogram of HSA, the spectrogram 44 illustrated in FIG. 5 is an example of the spectrogram of HDL, and the spectrogram 46 illustrated in FIG. 6 is an example of the spectrogram of LDL. In order to shorten the time required for the analysis, the frequency range and the time range for the analysis target may be narrowed down.

(3-2) Multivariable Analysis 1

Unscrambler X was started up, and all of the data (data set) acquired in the time-frequency analysis described above were read into this software. In addition, a data file name, a label for plot display at a later time, and a label for grouping process were input to the software. Then, primary component analysis (PCA) was executed on the data set. By reference to to the score plot which was generated, it was checked whether or not the samples were well separated. More specifically, a score plot was checked having an axis with the largest variance (first primary component axis (PC-1)), and an axis with the second largest variance (second primary component axis (PC-2)). When sufficient separation cannot be confirmed on the score plot, it is necessary to check other plots such as PC-3, PC-4, and the like. The loading distribution corresponding to the acquired score plot was checked, and was stored. The score plot 48 illustrated in FIG. 7 is an example of a score plot in Example 1.

(3-3) Loading Plot 1

The STFT tool was started up, one spectrogram generated through the time-frequency analysis was opened, and the loading distributions stored in the multivariable analysis 1 described above (the loading distribution of PC-1 and the loading distribution of PC-2) were read. Alternatively, the loading distributions of primary components of PC-3 and subsequent numbers may be read, while viewing the degree of separation and the contribution percentage. Using a particular loading distribution among the plurality of loading distributions which were read, a loading plot was generated and displayed. The time axis and the frequency axis in the loading plot corresponded to the time axis and the frequency axis in the spectrogram. In the loading plot, it was checked in what region the characteristic appeared. The loading plot 52 illustrated in FIG. 9 is an example of the loading plot of Example 1. Based on frequency information of the part where the characteristic appears, it is possible to investigate what component in the sample is related to the characteristic. In addition, based on time information of the part where the characteristic appears, it is possible to investigate from what property the characteristic is derived.

(3-4) Multivariable Analysis 2

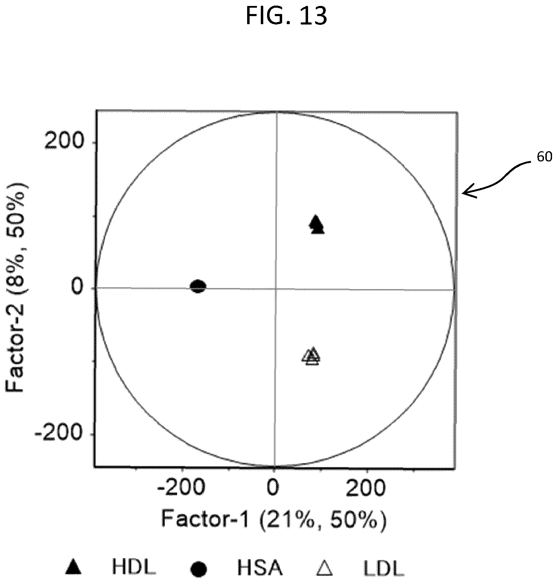

In multivariable analysis 2, in place of the primary component analysis (PCA), partial least squares discriminant analysis (PLS-DA) was executed. Unscrambler X was started up, and the partial least squares discriminant analysis (PLS-DA) was executed on the data set through a procedure similar to that for the multivariable analysis 1. FIG. 13 shows a score plot 60 generated by the partial least squares discriminant analysis (PLS-DA).

(3-5) Loading Plot 2

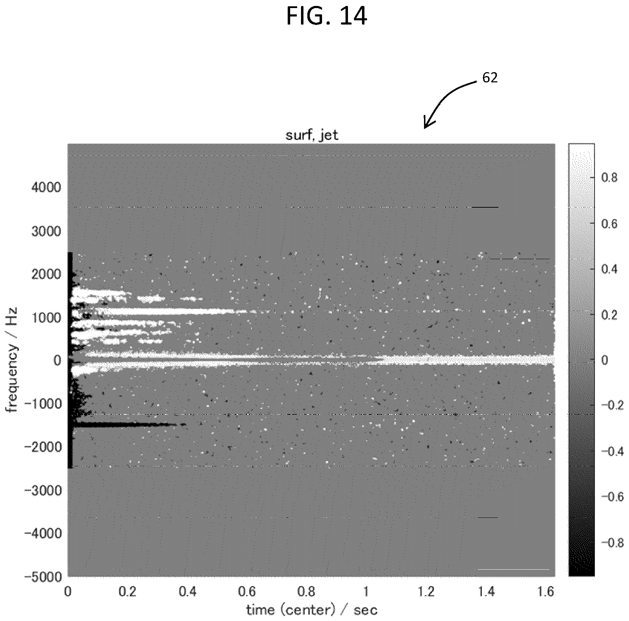

The STFT was started up, one spectrogram generated through the time-frequency analysis was opened through a procedure similar to that described above for the loading plot 1, and the loading distributions stored in the multivariable analysis 2 (the loading distribution of PC-1, and the loading distribution of PC-2) were read. A loading plot was generated using the loading distributions which were read, and the loading plot was displayed. In the loading plot, it was checked in what region the characteristic appeared. FIG. 14 shows this loading plot 62. Based on frequency information of a part where the characteristic appears, it is possible to investigate what component in the sample is related to the characteristic. Based on time information of the part where the characteristic appears, it is possible to investigate from what property the characteristic is derived.

In Example 1, it was possible to clearly separate the plurality of samples on the score plot. In addition, a difference could be observed among a plurality of frequency positions derived from a plurality of components included in HSA, a plurality of frequency positions derived from a plurality of components included in HDL, and a plurality of frequency positions derived from a plurality of components included in LDL. In other words, it was shown that the loading plot was useful as information for analyzing from what component in each sample the separation on the score plot was derived.

Example 2

An objective of Example 2 was executing a serum mode analysis on each of a diabetic model mouse (BKS. Cg db/db), and a healthy mouse (Jcl:ICR), and judging the onset of arteriosclerosis based on results of the analysis.

(1) Background and Objective

Diabetes causes onset of arteriosclerosis due to persistent hyperglycemia, and various cardiovascular events subsequent to the arteriosclerosis. Clinically, for close inspection of arteriosclerosis lesion, it is necessary to execute another inspection, other than the blood inspection. If it is possible to evaluate the arteriosclerosis lesion through a blood sample, such would be clinically useful. However, complicated molecular biological processes are associated with diabetes and subsequent arteriosclerosis lesion, and thus, it is not easy to detect all associated substances. Currently, no inspection method is established which enables detection and evaluation of the arteriosclerosis lesion from the blood sample of a diabetic patient. Analysis by NMR is an analysis method which enables evaluation of physiochemical characteristics of the serum. The inventors of the present disclosure postulated a hypothesis that it may be possible to use the analysis method to identify the blood state associated with progress of the arteriosclerosis lesion due to diabetes from healthy states, and analyzed a difference between the serum of a diabetic model mouse (BKS. Cg db/db) and the serum of a healthy mouse (Jcl:ICR). The diabetic model mouse (BKS. Cg db/db) is a mouse which exhibits a morbid state of the hyperglycemia from an early stage. The serum and the carotid tissues were collected from each of the diabetic model mouse (BKS. Cg db/db) and the healthy mouse (Jcl:ICR). Each serum was analyzed using the NMR apparatus, and each carotid tissue was pathologically inspected. It was reviewed whether or not early finding and progress evaluation of the morbid state associated with the arteriosclerosis lesion were possible from the NMR analysis results of the serum, based on two NMR analysis results and two pathological inspection results.

(2) Breeding of Experimental Animals

A diabetic model mouse (BKS. Cg db/db) and a healthy mouse (Jcl:ICR) (both purchased from CLEA Japan Inc.) were normally bred in a clean room in an experimental animal management room of Nippon Medical School. As the diet, general pellets (MF, manufactured by Oriental Yeast Co., Ltd.) were used, and the mice were free to eat and drink.

(3) Method of Sample Collection and Pathological Inspection

Maintenance anesthesia allowing sufficient analgesic effect by isoflurane was applied to the mouse, the chest of the mouse was opened, cardiac blood (about 1 mL) was collected, and the mouse was euthanized. After collecting the cardiac blood, the carotid tissue was collected in order to perform histologic inspection by HE stain. After collecting the carotid tissue, the carotid tissue was soaked in formalin solution, processes from embedding and subsequent processes, and HE stain were executed, and then, the carotid tissue was observed under a microscope.

The blood was centrifugally separated, to separate the serum. The separated serum; that is, a sample for NMR measurement, was stored at a temperature of -80°C until the time of NMR measurement.

Using a serum sample acquired from the mouse having a symptom of arteriosclerosis, operations and processes similar to those in Example 1 were performed, to generate a spectrogram, a score plot, and a loading plot for each serum sample.

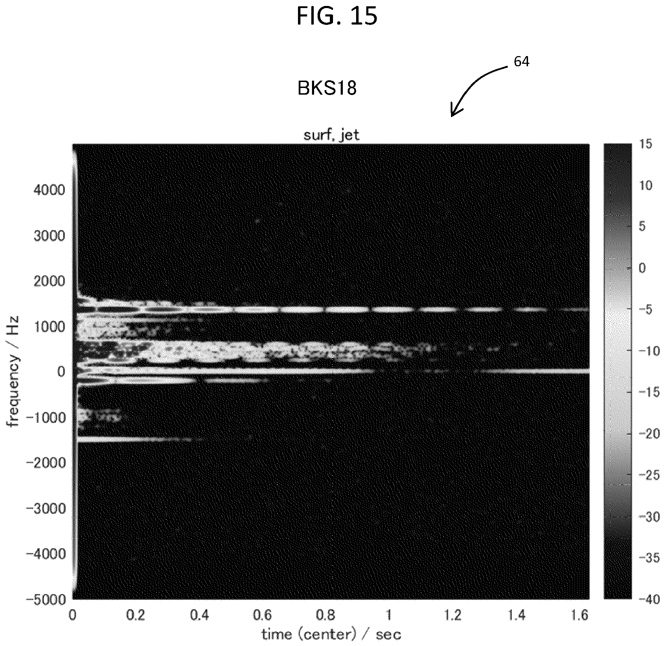

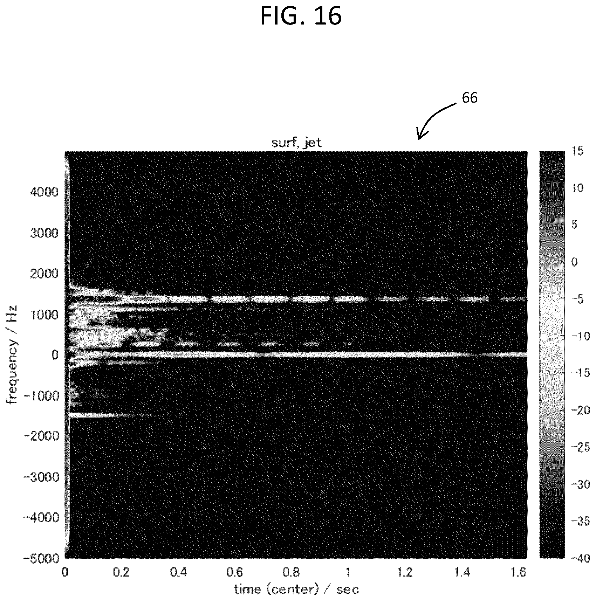

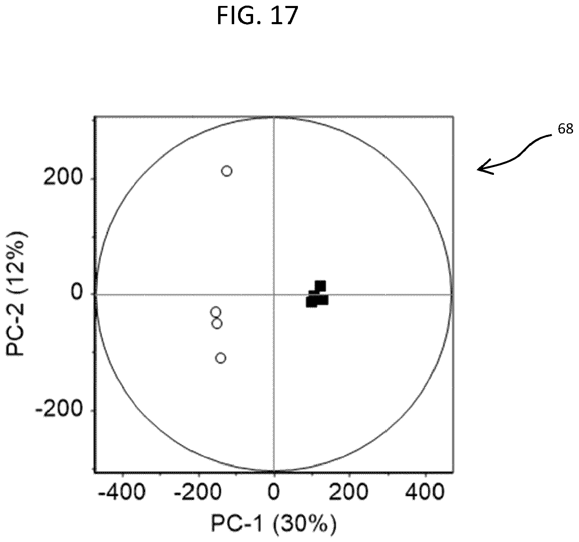

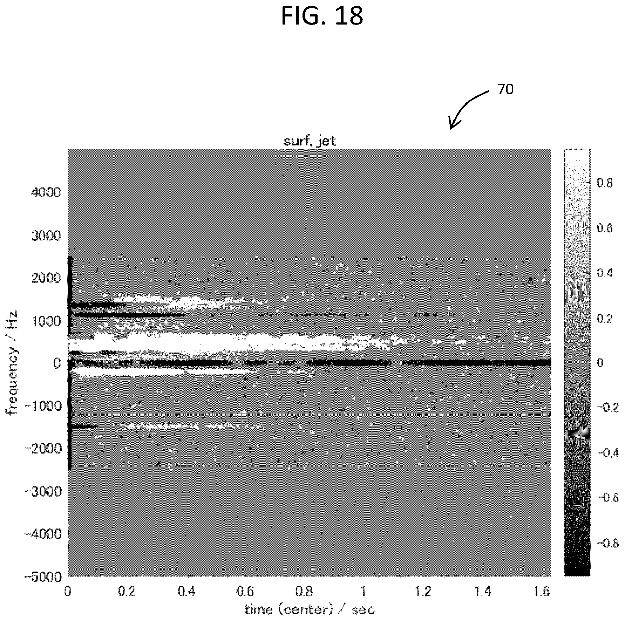

FIG. 15 shows a spectrogram 64 for BKS. FIG. 16 shows a spectrogram 66 for Jcl. FIG. 17 shows a score plot 68. The score plot 68 is a score plot generated by executing the primary component analysis (PCA). FIG. 18 shows a loading plot 70. The loading plot 70 is a loading plot generated based on the result of the primary component analysis (PCA).

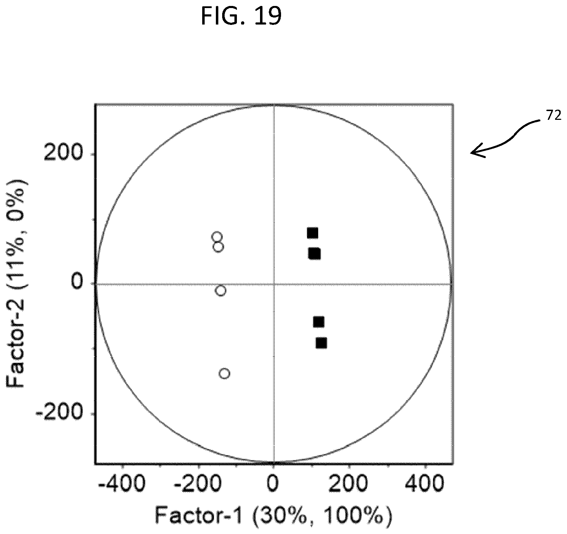

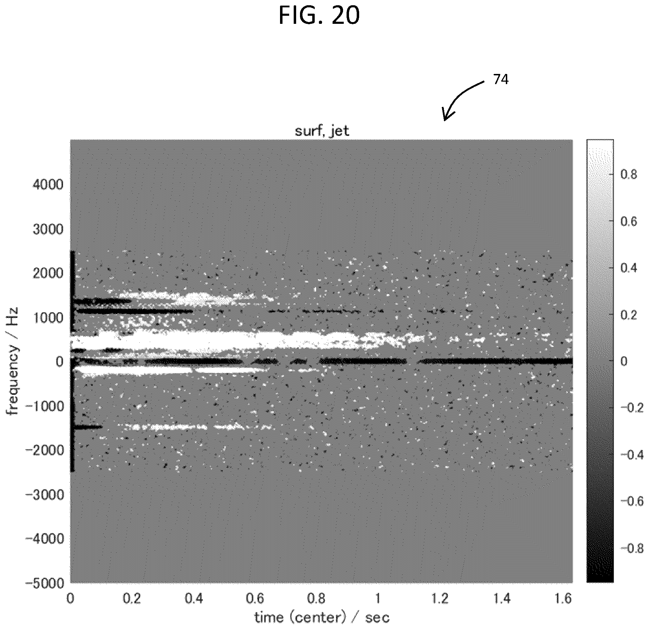

FIG. 19 shows another score plot 72. The score plot 72 is a score plot generated by the partial least squares discriminant analysis (PLS-DA). FIG. 20 shows a loading plot 74. The loading plot 74 is a loading plot generated based on a result of the partial least squares discriminant analysis (PLS-DA).

In a score plot of the sample with the symptom, a significant characteristic quantity (separation) was confirmed. When a factor which significantly affects the difference in the characteristic quantity was checked on the spectrogram, a large characteristic quantity was confirmed in signals which were derived respectively from HDL, LDL, and glucose. This result matches the medical viewpoint for factors for arteriosclerosis.

Result of Pathological Inspection

A pathological image of the carotid tissue was observed. For the healthy mice (Jcl:ICR), while a slight degree of increase in thickness of inner membrane due to obesity was observed in all mice, no presence of atheromatous plague was observed. For the diabetic model mice (BKS. Cg db/db), it was found that the degree of increase in the thickness of the inner membrane was higher in comparison to the healthy mouse (Jcl:ICR) of an age of 18 weeks and the atheromatous plague was found in more than half of the mice, indicating that arteriosclerosis was progressing in a short period at this age.
In Example 2, the samples could be clearly separated on the score plot. In addition, a difference in the frequency position derived from each component such as glucose could be read from the loading plot. This matches the medical viewpoint for causes of arteriosclerosis. That is, in Example 2 also, it was shown that the loading plot was useful as information for analyzing from what component in the sample the separation on the score plot was derived.

Example 3

An objective of Example 3 is judging a Parkinson's disease (PD) patient using a serum sample.

(1) Objective

Differentiating various disorders exhibiting Parkinsonism such as multiple system atrophy and progressive supranuclear palsy, and the Parkinson's disease (PD) is not necessarily easy at an early stage of onset, and no blood biomarker specific to the PD has yet been reported. Currently, as examples of markers which can identify, with the highest precision, PD and non-PD exhibiting Parkinsonism, there are known DAT-SPECT and MIBG myocardial scintigraphy. DAT-SPECT and MIBG myocardial scintigraphy were performed on PD patients and non-PD patients, and it was reviewed whether or not the PD and the non-PD could be identified by the serum sample using the NMR analysis developed by the inventors of the present disclosure. (2) Target
Under approval of the ethics committee, serums were collected from 10 patients who were medically treated in Nippon Medical School Chibahokusoh Hospital from October, 2020 to February 2022, and exhibiting any of the symptoms of tremor, hypokinesis, muscle regidity, and postural reflex impairment. A database was created based on blood biochemical data, head MRI data, MIGB myocardial scintigraphy data, and DAT-SPECT data. For 10 cases for which diagnosis and inspection were completed, NMR analysis was executed through a procedure similar to that in Example 1 on the serum collected from each of the patients. With this process, for each serum, a spectrogram, a score plot, and a loading plot were generated.

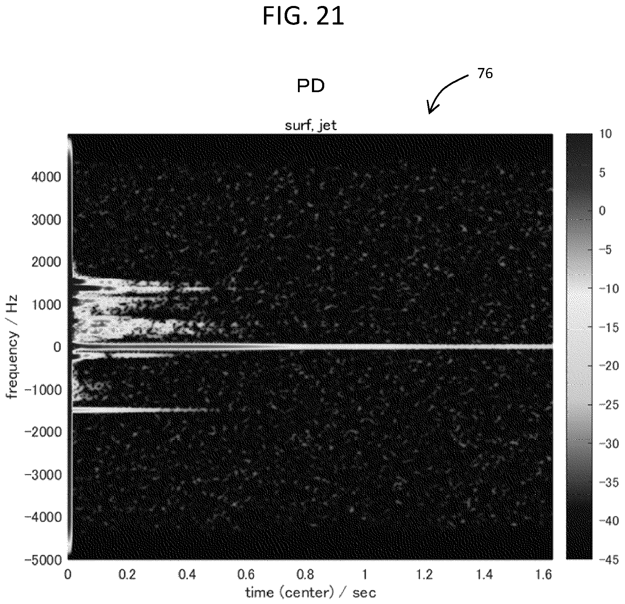

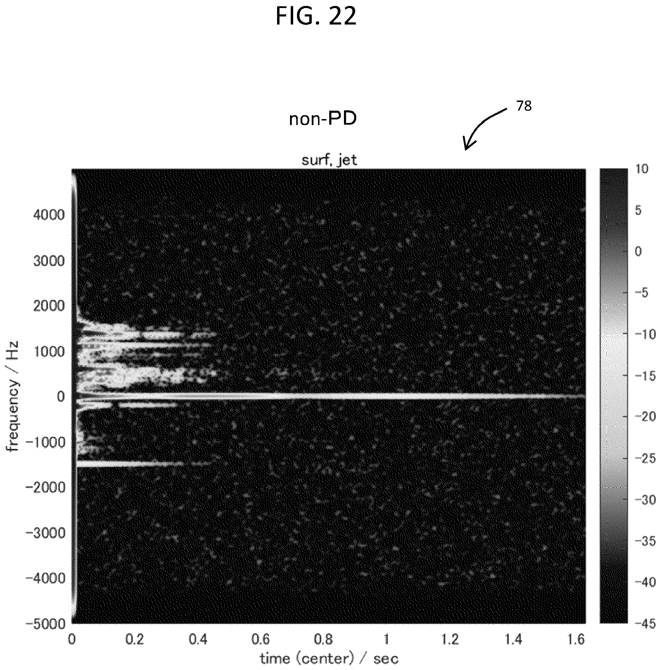

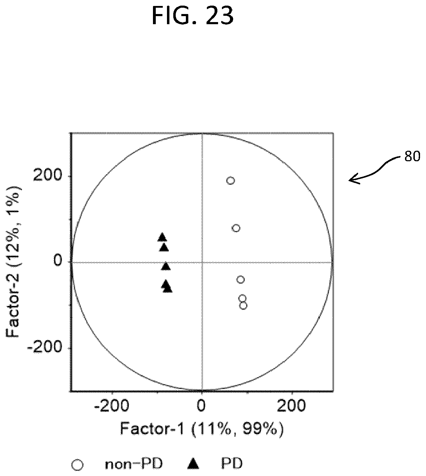

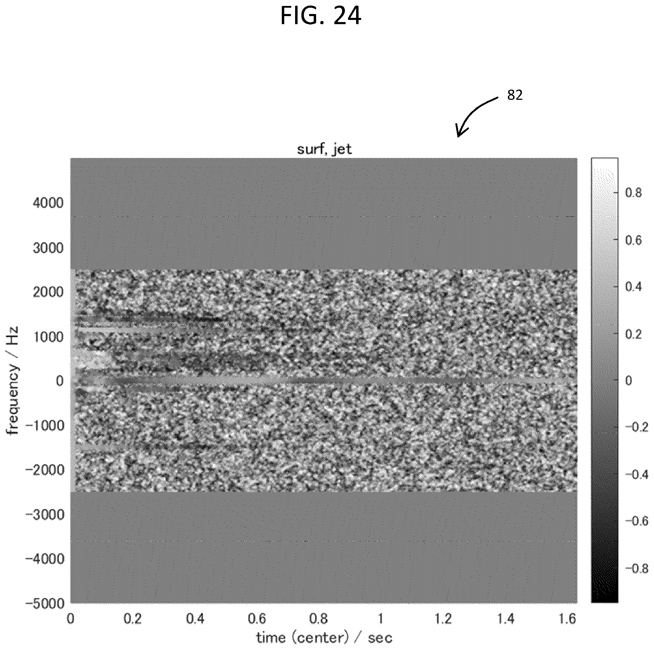

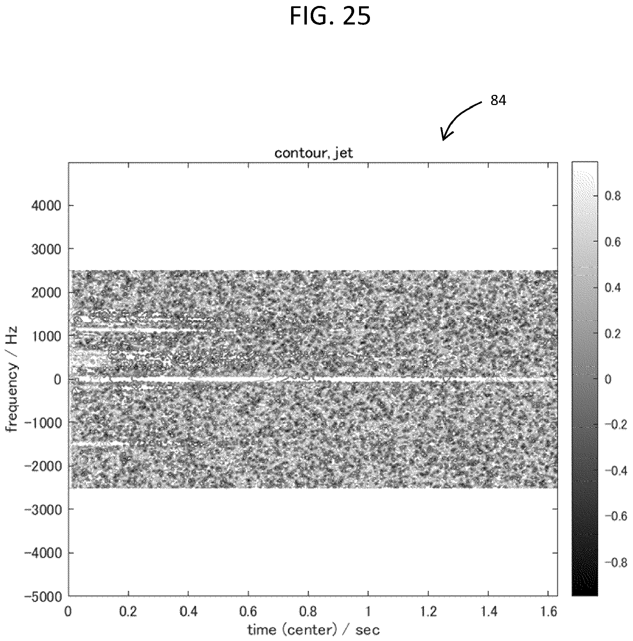

FIG. 21 shows a spectrogram 76 obtained from the serum of a PD patient. FIG. 22 shows a spectrogram 78 obtained from the serum of a non-PD patient. FIG. 23 shows a score plot 80. The score plot 80 is a score plot generated by the partial least squares discriminant analysis (PLS-DA). FIG. 24 shows a loading plot 82. FIG. 25 shows a loading plot 84. The loading plots 82 and 84 are loading plots generated based on the result of the partial least squares discriminant analysis (PLS-DA). In the loading plot 84, a contour line is displayed.

(3) Diagnosis

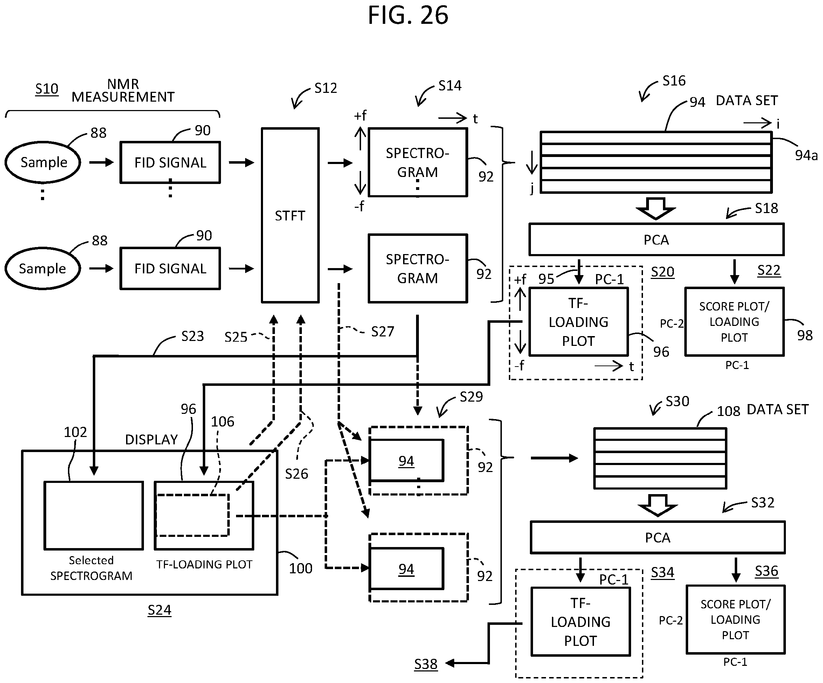

It was diagnosed that the patient is a PD patient when the conditions for "clinically definite PD" in the diagnosis standards (2015) of International Parkinson and Movement Disorder Society (MDS) were satisfied or when the conditions for "clinically probable PD" were satisfied. For the MIBG myocardial scintigraphy, abnormality was judged when the heart-to-mediastinum ratio of either an earlier phase or a later phase was less than or equal to 2.2. For the DAT-SPECT, visual evaluation and quantitative evaluation by SBR were executed, to check presence or absence or abnormality.
In Example 3, analysis based on PLS-DA was executed using the serum of the PD patient (having abnormal MIBG and abnormal DAT-SPECT), and the serum of the non-PD patient (having normal MIBG and normal DAT-SPECT). As a result, on the score plot, the groups formed clusters, and different groups were distributed in clearly different regions. Thus, possibility of identifying the PD patient and the non-PD patient through the NMR analysis of the serum was shown.
A data processing method (sample evaluation method) according to the embodiment will now be summarized with reference to FIG. 26. FIG. 26 shows a flow of a process executed by the processor.
In S10, NMR measurement is executed on a plurality of samples 88, and a plurality of FID signals 90 are thus acquired. In S12, short-time Fourier transform (STFT) is applied to the plurality of FID signals 90. In S14, for each FID signal 90, a spectrogram 92 is generated based on a sequence of frequency spectra generated by the short-time Fourier transform. That is, a plurality of spectrograms 92 corresponding to the plurality of samples 88 are generated. Each spectrogram 92 has a first coordinate system. In the first coordinate system, the horizontal axis is the time axis, and the vertical axis is the frequency axis.
In S 16, a data set 94 is formed based on the plurality of spectrograms 92. Specifically, the data set 94 is formed from a plurality of sub data sets 94a corresponding to the plurality of samples. Each sub data set 94a is formed from (mxn) intensities forming each spectrogram. The axis i shows an index axis, and the axis j shows a sample axis.

In S18, primary component analysis (PCA) is executed on the data set 94. In S22, a score plot 98 is generated as a result of the primary component analysis. In the illustrated example configuration, the score plot has a first primary component axis (PC-1) and a second primary component axis (PC-2). In S22, a general loading plot may be generated. For example, the general loading plot is generated based on a loading distribution corresponding to the first primary component, and a loading distribution corresponding to the second primary component, and has the first primary component axis (PC-1) and the second primary component axis (PC-2).

In S20, as a result of the primary component analysis, a loading plot (TF-loading plot) 96 of the embodiment is generated. For example, the loading plot 96 corresponding to the first primary component is generated based on a loading distribution 95 corresponding to the first primary component. The loading plot 96 has a second coordinate system. In the second coordinate system, the horizontal axis is the time axis and the vertical axis is the frequency axis. The second coordinate system is identical to the first coordinate system.

In S23, based on designation of a user, a particular spectrogram 102 is selected from among the plurality of generated spectrograms 92. In S24, the selected spectrogram 102 and the loading plot 96 are displayed on a display 100.

As necessary, in S25, a parameter set for the short-time Fourier transform is changed by the user by reference to the loading plot 96. For example, the frame length is changed. In this case, the short-time Fourier transform is re-executed on the plurality of FID signals 90 in accordance with the changed parameter set. The primary component analysis is applied to a new data set formed from a plurality of new spectrograms generated through this process.

In addition, as necessary, an analysis region 106 is set on the loading plot 96. In this case, in S26, under a transformation condition according to the analysis region 106, the short-time Fourier transform is re-executed on the plurality of FID signals 90. A limited data set is formed by a plurality of new spectrograms generated by the re-execution of the short-time Fourier transform. The primary component analysis is applied on the limited data set. Alternatively, the parameter set may be changed prior to execution of S26.

When the analysis region 106 is set on the loading plot 96, a plurality of parts 94 corresponding to the analysis region 106 may be cut out from the plurality of spectrograms 92 which are already generated, without re-executing the short-time Fourier transform. Specifically, in S27, the plurality of spectrograms 92 which are already generated are acquired, and in S29, parts 94 corresponding to the analysis region 106 are cut out from the acquired spectrograms 92. In S30, a limited data set 108 is formed from the plurality of cut-out parts 94. In S32, the primary component analysis is applied on the limited data set 108. With this process, for example, in S36, a score plot and/or a general loading plot is generated as a result of the primary component analysis. In S34, a loading plot of the embodiment is generated based on the loading distribution generated by the primary component analysis. The loading plot is generated, for example, from the loading distribution corresponding to the first primary component. Then, in S38, the loading plot is displayed as necessary. An analysis region may be set on the loading plot. In this case, the sequence of processes described above are re-executed.

As described, according to the present embodiment, the content of the spectrogram and the content of the loading plot can be investigated while comparing the spectrogram and the loading plot. In this manner, factors which cause differences in characteristics and attributes among a plurality of samples can be analyzed. As a result, for example, in the medical field, realization of preemptive medicine (that is, a medical care in which a disorder is predicted before a symptom appears, a therapeutic intervention is made, and onset of the disorder is prevented or delayed) can be expected. For example, realization of very early diagnosis, determination of therapeutic plans, judgement of therapeutic effect, and prognostic prediction can be expected. In addition, according to the present embodiment, there is a possibility that it becomes possible to search cases or the like without the use of known attribute identification images.

## Claims

1. A data processing apparatus comprising:

   acquisition means (14) that acquires a plurality of spectrograms generated by executing NMR measurement on a plurality of samples, each of the plurality of spectrograms having a first coordinate system with a time axis and a frequency axis;
   analysis means (18, 20) that executes multivariable analysis on a data set formed from the plurality of spectrograms, to identify a primary component of the data set and generate a loading distribution corresponding to the primary component, the loading distribution being formed from a plurality of loadings corresponding to a plurality of variables in the multivariable analysis; and
   generation means (22) that generates a loading plot having a second coordinate system with a time axis and a frequency axis, based on the loading distribution, wherein
   the second coordinate system is identical to the first coordinate system.

2. The data processing apparatus according to claim 1, wherein

a particular spectrogram is selected from among the plurality of spectrograms, and

the data processing apparatus further comprises display control means (26) that causes the particular spectrogram and the loading plot to be displayed side by side on a display.

3. The data processing apparatus according to claim 1 or 2, further comprising:

setting means (31) that sets, on the loading plot, an analysis region defined by at least one of a time range or a frequency range, wherein
the analysis means (18, 20) re-executes the multivariable analysis on a limited data set corresponding to the analysis region.

4. The data processing apparatus according to claim 3, wherein

in the loading plot, the time range and the frequency range are designated by a user, and
the setting means (31) sets the analysis region in accordance with the time range and the frequency range designated by the user.

5. The data processing apparatus according to claim 3, wherein
the setting means (31) sets, as the analysis region, a region which belongs to the time range and the frequency range, and which satisfies a particular loading condition.

6. The data processing apparatus according to any preceding claim, wherein
the generation means (22) generates the loading plot through coordinate transformation of the loading distribution.

7. The data processing apparatus according to any one of claims 3 to 6, further comprising:
means (S29) that cuts out the limited data set from the data set based on the analysis region.

8. The data processing apparatus according to any one of claims 3 to 7, further comprising:

means (16, S12) that executes time-frequency analysis on a plurality of FID signals which are generated by executing the NMR measurement on the plurality of samples in accordance with a parameter set, to thereby generate the plurality of spectrograms;
means (S26) that corrects the parameter set based on the analysis region; and
means (16, S12) that re-executes the time-frequency analysis on the plurality of FID signals in accordance with the corrected parameter set, to thereby generate a plurality of spectrograms forming the limited data set.

9. A program for executing a data processing method in a computer, the data processing method comprising:

an acquisition step (14) of acquiring a plurality of spectrograms generated by executing NMR measurement on a plurality of samples, each of the plurality of spectrograms having a first coordinate system with a time axis and a frequency axis;
an analysis step (18, 20, S18) of executing multivariable analysis on a data set formed from the plurality of spectrograms, to identify a primary component of the data set and generate a loading distribution corresponding to the primary component, the loading distribution being formed from a plurality of loadings corresponding to a plurality of variables in the multivariable analysis; and
a generation step (22, S20) of generating a loading plot having a second coordinate system with a time axis and a frequency axis, based on the loading distribution, wherein
the second coordinate system is identical to the first coordinate system.

10. A method of evaluating a sample, comprising:

an acquisition step (14) of acquiring a plurality of spectrograms generated by executing NMR measurement on a plurality of samples, each of the plurality of spectrograms having a first coordinate system with a time axis and a frequency axis;
an analysis step (18, 20, S18) of executing multivariable analysis on a data set formed from the plurality of spectrograms, to identify a primary component of the data set and generate a loading distribution corresponding to the primary component, the loading distribution being formed from a plurality of loadings corresponding to a plurality of variables in the multivariable analysis; and
a generation step (22, S20) of generating a loading plot having a second coordinate system with a time axis and a frequency axis, based on the loading distribution, wherein
the second coordinate system is identical to the first coordinate system.

# FIG. 1

EP 4 478 073 A1

NMR
APPARATUS
10

DATA PROCESSING APPARATUS
12

| RECEIVER | FREQUENCY ANALYZER | MULTIVARIABLE ANALYZER | DISTRIBUTION GENERATOR | PLOT GENERATOR |
|---|---|---|---|---|
| 14 | 16 | 18 | 20 | 22 |

| STORAGE | DISPLAY CONTROLLER | DISPLAY UNIT | MANIPULATION UNIT | SPECIFIER |
|---|---|---|---|---|
| 24 | 26 | 28 | 30 | 31 |

# FIG. 2

DATA PROCESSING APPARATUS 20

COMMUNICATION DEVICE 32

UI 34

STORAGE DEVICE 36

PROCESSOR 38

# FIG. 3

amplitude

40

time

EP 4 478 073 A1

FIG. 4

42

HSA

surf, jet

EP 4 478 073 A1

EP 4 478 073 A1

FIG. 5

HDL

surf, jet

44

# FIG. 6

LDL

surf, jet

FIG. 7

EP 4 478 073 A1

FIG. 8

FIG. 9

surf, jet

52

FIG. 10

54    56

n

m    1,1   1,2        k    1   4
      2,1   2,2             2   5
      3,1   3,2             3   6

EP 4 478 073 A1

FIG. 11

FIG. 12

FIG. 13

FIG. 14

surf, jet

EP 4 478 073 A1

# FIG. 15

BKS18

64

surf, jet

# FIG. 16

EP 4 478 073 A1

FIG. 17

EP 4 478 073 A1

FIG. 18

surf.jet

70

time (center) / sec

frequency / Hz

FIG. 19

# FIG. 20

74

surf, jet

EP 4 478 073 A1

## FIG. 21

PD

76

surf, jet

frequency / Hz

time (center) / sec

## FIG. 22

FIG. 23

FIG. 24

FIG. 25

84

contour, jet

EP 4 478 073 A1

# FIG. 26

EP 4 478 073 A1

Europäisches
Patentamt

European
Patent Office

Office européen
des brevets

# EUROPEAN SEARCH REPORT

Application Number

EP 24 17 9890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HIRAKAWA KEIKO ET AL: "Short-time Fourier Transform of Free Induction Decays for the Analysis of Serum Using Proton Nuclear Magnetic Resonance", JOURNAL OF OLEO SCIENCE, vol. 68, no. 4, 1 January 2019 (2019-01-01), pages 369-378, XP093219555, JP ISSN: 1345-8957, DOI: 10.5650/jos.ess18212 * Chapter 1: Introduction, Chapter 2: Experimental; figures 1-5 * | 1-10 | INV. G01R33/46 G01R33/465 |
| A,D | WO 2015/087892 A1 (UNIV KYOTO [JP]; NIPPON MEDICAL SCHOOL FOUNDATION [JP] ET AL.) 18 June 2015 (2015-06-18) * the whole document * | 1-10 | |
| X,P | YUI KANAKO ET AL: "Time-frequency analysis reveals an association between the specific nuclear magnetic resonance (NMR) signal properties of serum samples and arteriosclerotic lesion progression in a diabetes mouse model", PLOS ONE, vol. 19, no. 3, 8 March 2024 (2024-03-08), page e0299641, XP093219633, US ISSN: 1932-6203, DOI: 10.1371/journal.pone.0299641 * the whole document * | 1-10 | **TECHNICAL FIELDS SEARCHED (IPC)** G01R G01N |

The present search report has been drawn up for all claims

| Place of search | Date of completion of the search | Examiner |
|---|---|---|
| Munich | 31 October 2024 | Faber-Jurk, Sonja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 9890

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2024

| Patent document cited in search report | Publication date | Patent family member(s) | Publication date |
|---|---|---|---|
| WO 2015087892 A1 | 18-06-2015 | JP 6281973 B2 | 21-02-2018 |
| | | JP 2015114157 A | 22-06-2015 |
| | | WO 2015087892 A1 | 18-06-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015114157 A
- JP 2019158868 A
- JP 5415476 B
- JP 5020491 B